# EUROPEAN PATENT APPLICATION

(11) **EP 4 368 708 A1**
(43) Date of publication of application: **15.05.2024**
(21) Application number: 22831198.1
(22) Date of filing: 24.02.2022
(51) Int. Cl.: C12N 9/00, C12N 15/52, C12P 19/34

(54) **NUCLEIC ACID LIGASE**

(30) Priority: 01.07.2021 CN 202110754255
(71) Applicant: Wenzhou Medical University, Wenzhou, Zhejiang 325000 (CN)
(72) Inventor: DING, Chunming, Wenzhou, Zhejiang 325000 (CN); YANG, Zhengquan, Wenzhou, Zhejiang 325000 (CN); JIN, Shengnan, Wenzhou, Zhejiang 325000 (CN)
(74) Representative: Patentanwälte Ruff, Wilhelm, Beier, Dauster & Partner mbB
(86) International application number: PCT/CN2022/077538
(87) International publication number: WO 2023/273366

(57) **Abstract**

Provided is a nucleic acid ligase, which comprises an amino acid sequence having a mutation at one or more positions selected from positions 79, 281, 370 and 372 compared to the amino acid sequence of Hyperligase of the prior art (SEQ ID NO: 1). Also provided are a nucleic acid molecule encoding the enzyme, a vector comprising the nucleic acid molecule, and a recombinant cell comprising the nucleic acid molecule or the vector. Also provided are a composition containing the enzyme and a use of the enzyme.

## Description

### Technical field

The present invention relates to the field of biotechnology. Specifically, it relates to new nucleic acid ligases obtained by mutating a nucleic acid ligase using current techniques. The invention also relates to a product comprising of said new enzymes.

### Background

Nucleic acid ligases are metal ion-dependent enzymes that catalyze the formation of a phosphodiester bond between the adjacent 3' end and 5' end of DNA or RNA molecules to achieve intramolecular cyclization or intermolecular linear ligation of a substrate sample. Nucleic acid ligases can be divided into DNA ligases and RNA ligases according to their catalytic substrates. Some ligases can catalyze the ligation reactions between DNA and RNA ^{[1, 2]}. Depending on what species they are derived from, natural nucleic acid ligases have a variety of unique properties, such as substrate specificity, sequence preference, thermal stability, salt tolerance, pH change tolerance, etc.

It is generally believed that a complete nucleic acid ligation reaction is divided into three steps ^{[1]}. In the first step, the ligase binds to the adenosine group of ATP or NAD⁺, transfers the adenosine group in ATP or NAD⁺ to a lysine residue in a conserved motif of the enzyme, releases a pyrophosphate, and forms an intermediate between the enzyme and the adenosine. This step is an equilibrium reaction and can proceed in both directions. In the second step, the intermediate between the enzyme and the adenosine transfers its adenosine group to the 5' phosphate end of a nucleic acid substrate to form a 5' adenylated intermediate. This step is also an equilibrium reaction and can proceed in both directions. In the third step, the lysine catalytic site of the enzyme binds to the 3' hydroxyl end of the nucleic acid, attacks the 5' adenylated end of the nucleic acid, catalyzes the formation of a phosphodiester bond between the two, and completes the ligation reaction. Common ligases generally use 5' phosphorylated nucleic acids as substrates to catalyze all three steps of the ligation reaction to complete the ligation reaction ^{[3, 4]}.

Ligases are important tools in molecular biology research and molecular diagnostic technology since they are the basis of common molecular biology methods such as molecular cloning technology, high-throughput sequencing library preparation, gene synthesis and molecular diagnosis ^{[5, 6]}. According to the reaction temperature of the enzymes, ligases can be divided into two types: thermostable and non-thermostable. Currently, there are many ligases used for doublestranded DNA or RNA ligation reactions, but there are extremely limited choices for enzymes used for single-stranded DNA ligation reactions. There are even fewer thermostable ligases which can perform single-stranded ligation at higher temperatures (such as >65°C).

Several thermostable single-stranded nucleic acid ligases have been reported. The thermostable TS2126 ligase (trade name: CircLigase) can directly catalyze the ligation reaction between 5' phosphate end and 3' hydroxyl end at 65°C^{[7]}, but the catalysis has end sequence preference, resulting in selective ligation of the substrates^{[7]}, which leads to bias in the results. The genetically mutated Mth RNA thermostable ligase (trade name: Thermostable 5'AppDNA&RNA Ligase) can also catalyze the ligation reaction between single-stranded nucleic acids at higher temperatures, but its ligation efficiency is lower when used in single-stranded DNA ligation reactions ^{[8]}. Taq DNA ligase, another enzyme that can catalyze the ligation reaction between single-stranded DNAs at higher temperatures, has higher ligation efficiency, but it requires the complementary strand as a template for guidance, and cannot be used alone in the ligation reaction of single-stranded nucleic acids.

Engineering and modification of enzymes mainly include genetic mutation, gene fusion, chemical modification, antibody modification, and nucleic acid aptamer modification, etc. Genetic engineering of some ligases can change the characteristics of the ligases such as improving the thermal stability or ligation efficiency, and the salt tolerance or acid tolerance. Mutation of the core lysine of the Mth RNA ligase allows it to directly use pre-adenylated single-stranded DNAs as substrates to perform ligation reactions to improve its ligation efficiency ^{[8]}. Genetic mutation of the core lysine of the protein encoded by the *Hbut_1550* gene from *Hyperthermus butylicus* confers the protein the ability to ligate single-stranded DNA/RNAs, so researchers engineered it into a heat-stable DNA/RNA Ligase (named Hyper-Thermostable Lysine-Mutant ssDNA/RNA Ligase, hereinafter referred to as HyperLigase, WO2017160788A3). HyperLigase ssDNA/RNA Ligase has high thermal stability and can catalyze the ligation reaction between the 5' pre-adenylated substrate and the 3' hydroxyl end in a wider temperature range (such as 37-95°C). Despite this, the genetically engineered HyperLigase still suffer from insufficient ligation efficiency.

Therefore, there is still a need in the art for thermostable single-stranded nucleic acid ligases with higher ligation efficiency.

### Summary of the invention

Based on a previous invention, by screening potential genetic mutation sites and carrying out genetic engineering, HyperLigase DNA/RNA ligases with new mutation sites were obtained in the present invention. Compared with the original HyperLigase DNA/RNA Ligase, these modified HyperLigase DNA/RNA ligases have higher enzymatic activities and improved catalytic activities.

Specifically, the present invention relates to the following aspects:
In one aspect, the present invention relates to a nucleic acid ligase, which comprises an amino acid sequence having mutations at one or more positions selected from positions 79, 281, 370 and 372 compared to the amino acid sequence of SEQ ID NO: 1. In one embodiment, the mutation at position 79 is the substitution of Arg with Ala, the mutation at position 281 is the substitution of Arg with Ala, the mutation at position 370 is the substitution of Lys with any natural amino acid except Lys, and the mutation at position 372 is the substitution of Lys with any natural amino acid except Lys. In one embodiment, the mutation at position 372 is the substitution of Lys with Glu, Cys, Val, Ser, Gln, Ala, Leu, Thr or Phe.

In one embodiment, the nucleic acid ligase of the invention comprises an amino acid sequence selected from SEQ ID NOs: 2-7.

In one embodiment, the nucleic acid ligase of the invention comprises the amino acid sequence of SEQ ID NO: 2 or 3, wherein Xaa is a non-polar amino acid, preferably Ala. In one embodiment, the nucleic acid ligase of the invention comprises the amino acid sequence of SEQ ID NO: 4 or 5.

In one embodiment, the nucleic acid ligase of the invention comprises the amino acid sequence of SEQ ID NO:6. In one embodiment, Xaa at positions 370 and 372 of SEQ ID NO: 6 is Ala.

In one embodiment, the nucleic acid ligase of the present invention comprises the amino acid sequence of SEQ ID NO: 7, wherein Xaa at positions 79 and 281 is a non-polar amino acid, preferably Ala. In one embodiment, Xaa at positions 370 and 372 of SEQ ID NO: 7 is a non-polar amino acid, preferably Ala.

In another aspect, the invention relates to a nucleic acid molecule encoding the nucleic acid ligases of the invention.

In another aspect, the invention relates to a vector comprising a nucleic acid molecule encoding the nucleic acid ligase of the invention.

In another aspect, the invention relates to recombinant cells into which the nucleic acid of the invention or the vector of the invention has been introduced.

In another aspect, the invention relates to a composition for ligating single-stranded DNAs and/or RNAs, which comprises a nucleic acid ligase of the invention.

In another aspect, the present invention relates to a kit for ligating single-stranded DNAs and/or RNAs, which comprises the nucleic acid ligase of the present invention.

In another aspect, the invention relates to use of a nucleic acid ligase of the invention in the preparation of a product for ligating single-stranded DNAs and/or RNAs.

### Brief description of the drawings

Figure 1 shows the electrophoretic analysis of the mutant HyperLigase gene fragment amplified by inverse PCR (taking part of the results as an example).
Figure 2 shows the capillary sequencing results for confirmation of wild-type and mutant HyperLigases (taking the K370C site as an example).
Figure 3 shows the SDS-PAGE electrophoresis analysis of the purified mutant HyperLigase (taking part of the results as an example).
Figure 4 shows the linear ligation reaction activity comparison among mutant HyperLigase, wild-type HyperLigase and CircLigase. Each data is the average of three independent replicate experiments; T test; *: p<0.05; **: p<0.01, ***: p<0.001.
Figure 5 shows the comparison of the single-stranded cyclization reaction activities of mutant HyperLigase and wild-type HyperLigase. Each data is the average of three independent replicate experiments; T test; *: p<0.05; **: p<0.01, ***: p<0.001.
Figure 6 shows the comparison of the linear ligation activities of mutant HyperLigase and wild-type HyperLigase. Each data is the average of three independent replicate experiments; T test; *: p<0.05; **: p<0.01, ***: p<0.001.
Figure 7 shows a summary of the cyclization efficiency of all mutant Hyperligases. The figure shows a summary of the cyclization efficiencies of all mutant Hyperfigases ranked from high to low; each data is the average of three independent replicate experiments; T test; *: p<0.05; **: p <0.01, ** *: p<0.001.
Figure 8 shows a summary of the linear ligation efficiency of all mutant Hyperligases. The figure shows a summary of the linear ligation efficiencies of all mutant Hyperfigases ranked from high to low; each data is the average of three independent replicate experiments; T test; *: p<0.05; **: p <0.01, ** *: p<0.001.
Figure 9 compares the activities between the wild-type Hyperligase and the K370P mutant on the ligation between 3' hydroxyl end of RNA and the 5' adenylated end of DNA, showing that the K370P mutation enhances the activity of Hyperligase on the ligation between 3' hydroxyl end of RNA and the 5' adenylated end of DNA.
Figure 10 compares the activities between the wild-type Hyperligase and the K370P mutant on the ligation between 3' hydroxyl end of DNA and the 5' adenylated end of RNA, showing that the K370P mutation enhances the activity of Hyperligase on the ligation between 3' hydroxyl end of DNA and the 5' adenylated end of RNA.

### Detailed description of the invention

The following examples are provided to show preferred embodiments. Those skilled in the art will recognize that the technologies disclosed in the following examples represent those discovered by the inventors to function well in the practice of the methods disclosed herein, and thus may be considered to constitute preferred modes of practice. In light of the present disclosure, however, those skilled in the art will recognize that many changes can be made in the specific embodiments disclosed without departing from the principles and scope of the methods disclosed herein and still obtain the same or similar results.

### Example 1.

Construction of a protein expression plasmid with mutant HyperLigase using site-directed mutagenesis

Starting from the HyperLigase of the prior art (SEQ ID NO: 1), the applicant conducted analysis and research and screened out potential mutation sites, namely Arg79, Lys249, Lys370 and Lys372 of HyperLigase.

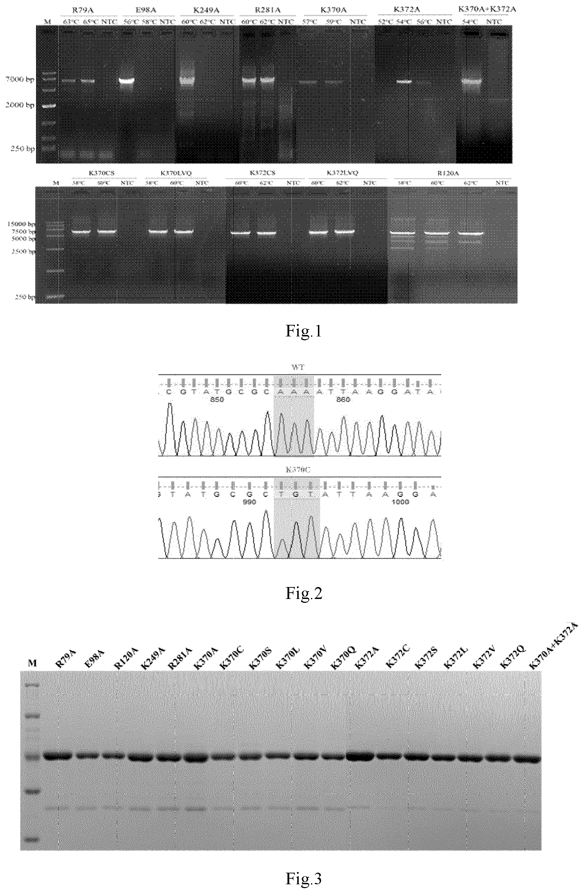

Based on the screened mutation sites and the corresponding amino acids to be modified, the NEBasechanger site-directed mutation primer online design tool (https://nebasechanger.neb.com) was used to design back-to-back point mutation primers. After synthesizing the corresponding primers, the wild-type HyperLigase protein (SEQ ID NO: 1) expression plasmid was used as a template and a high-fidelity DNA polymerase was used to perform a PCR reaction. After the product was analyzed by electrophoresis to have the correct size, fragment purification was performed (Figure 1). After purification, the DNA was terminally phosphorylated and circularized. The product was transformed into BL21 (DE3) competent cells and plated on a plate containing antibiotics. The colonies obtained by antibiotic screening were selected and sequenced to confirm that the sequence was correct (see Figure 2). Mutant HyperLigase protein expression plasmids were obtained. The above procedures all used commercial reagents and were performed in accordance with the instructions of the kit. HyperLigase protein was expressed with a His tag located at the N-terminus or C-terminus of the target protein.

In addition to the above single mutations, two sites, K370 and K372, were selected for co-mutation to construct a double mutated K370A+K372A enzyme in which both sites were simultaneously mutated to alanine.

### Example 2.

### Expression induction and purification of the proteins

Positive colonies were picked from the plate and were inoculated into LB medium for recovery. After recovery, the culture was expanded by 10 to 50 times and then transferred to one Liter LB medium and cultured until the OD600 absorbance was 0.6 to 0.8, so that the bacteria reached the logarithmic growth phase. IPTG inducer was added to a final concentration of 0.1 to 1 mmol/L, and the culture was induced and cultured at 14 to 37°C for 6 to 24 hours. The bacterial cells were collected and precipitated, and then were broken by ultrasonic treatment, and the broken products were purified. Purification was carried out using manual or instrumental methods, following the instructions for the equipment's. After elution, the protein concentration was measured and converted to molar concentration, and SDS-PAGE was used for purity identification (Figure 3). The mutant HyperLigases were stored in 50% glycerol solution at -20°C for long-term storatge.

### Example 3.

### Comparison of activities among mutated HyperLigase enzymes, the original Hyperligase and CircLigase enzymes

CircLigase is the most active single-stranded DNA ligase currently known on the market. In order to test the activities of HyperLigases before and after mutation, and CircLigase, an intermolecular linear ligation reaction was used to compare the activities of the enzymes.

The CircLigase linear ligation reaction system was as follows:

| reagent | Final concentration | 1× (µL) |
|---|---|---|
| 10×Circligase II Buffer | 1× | 2 |
| 50 mM MnCl₂ | 2.5 mM MnCl₂ | 1 |
| 5'-rApp/3'-blocked oligo | 0.5 µM | 1 |
| 3'-OH oligo | 0.1 µM | 1 |
| PEG8000,50% w/v | 20% | 8 |
| CircLigase | 50 U | 0.5 |
| H₂O | / | To 20µL |

The HyperLigases linear ligation reaction system was as follows:

| reagent | Final concentration | 1× (µL) |
|---|---|---|
| 10×HyperLigase Buffer, pH=7.5 | 70 mM Tris-HCl | 1 |
| 20×MnCl₂ | 10 mM MnCl₂ | 0.5 |
| 5'-rApp/3'-blocked oligo | 0.5 µM | 1 |
| 3'-OH oligo | 0.1 µM | 1 |
| PEG8000,50% w/v | 20% | 8 |
| Wild-type or mutant HyperLigase | 0.5 µM | 1 |
| H₂O | | To 20 µL |

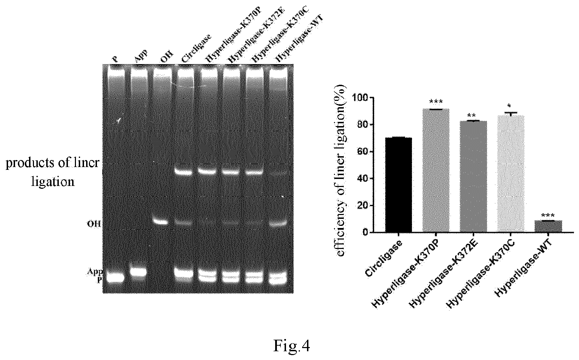

The HyperLigase reaction was carried out at 75°C, and the CircLigase reaction was carried out at 60°C. The reaction time was 6 hours for both. At the end of the reaction, Urea-PAGE electrophoresis and grayscale analysis were carried out to compare the difference in enzyme activities (Figure 4).

The test results showed that the K370P mutation, K372E mutation, and K370C mutation all significantly improved the catalytic activity of HyperLigase, and the activity of HyperLigase after mutation was significantly higher than that of CircLigase.

### Example 4.

### Activity test of mutant HyperLigase enzymes

### (1) Circularization efficiency test

Using the synthesized pre-adenylated substrates, the intramolecular circularization reaction system was prepared according to the following table:

| reagent | Final concentration | 1× (µL) |
|---|---|---|
| 10×HyperLigase Buffer, pH=7.5 | 70 mM Tris-HCl | 1 |
| 20×MnCl₂ | 10 mM MnCl2 | 0.5 |
| 5'rApp oligo | 0.1 µM | 1 |
| Wild-type or mutant HyperLigase | 0.1 µM | various |
| H₂O | | To 10 µL |

The reaction conditions were 60 to 75°C for 6 hours, and maintained at 4°C.

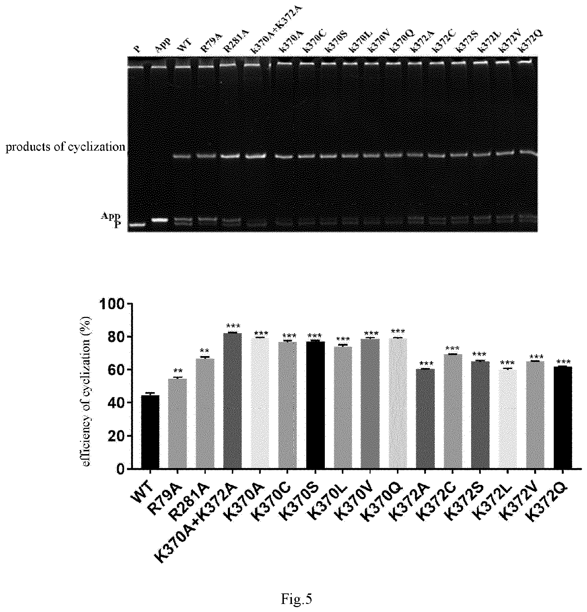

Reaction product detection: The circularized products were identified by Urea-PAGE electrophoresis. The catalytic activities of the enzymes were characterized according to the change in the positions of the electrophoresis bands. The difference in the catalytic activities of the enzymes were compared according to the ratio of the grayscale values of the circularized product band to the substrate band as catalyzed by different mutant enzymes. The results are shown in Figure 5.

The results of the single-stranded DNA circularization reaction showed that the R79, R281, K370, or K372 to alanine mutants significantly improved the activity, and the double mutant of K370 and K372 to alanine improved the catalytic activity. At the same time, for positions K370 and K372, mutation of lysine to other amino acids all improved the catalytic activity.

### (2) Linear ligation efficiency test

Using a synthetic substrate with adenylation at the 5' end and a blocking modification at the 3' end, and a substrate with a hydroxyl group at the 3'end, a linear ligation reaction system was prepared according to the following table:

| reagent | Final concentration | 1× (µL) |
|---|---|---|
| 10×HyperLigase Buffer, pH=7.5 | 70 mM Tris-HCl | 2 |
| 20×MnCl₂ | 10 mM MnCl₂ | 1 |
| 5'-rApp/3'-blocked oligo | 0.5 µM | 1 |
| 3'-OH oligo | 0.1 µM | 1 |
| PEG8000, 50% w/v | 20% | 8 |
| Wild-type or mutant HyperLigase | 0.5 µM | various |
| H₂O | / | To 20 µL |

The reaction conditions were 60 to 75°C for 6 hours, and then maintained at 4°C.

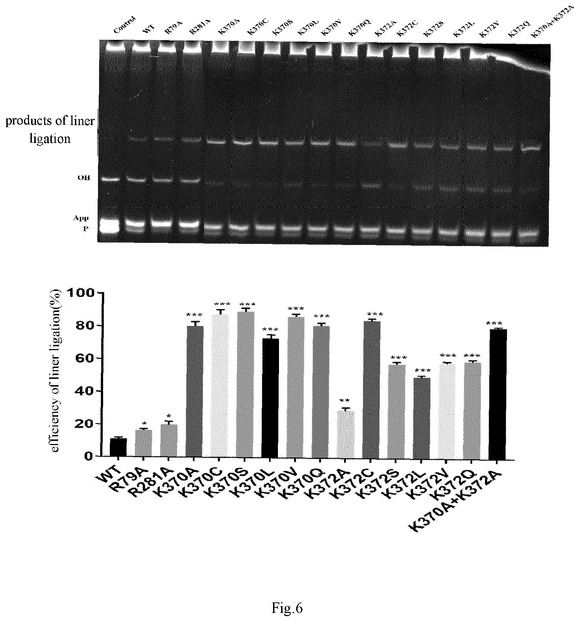

Reaction product detection: The linear ligation product was analyzed by Urea-PAGE electrophoresis. The catalytic activities of the enzymes were characterized according to the change in the positions of the electrophoresis band. The difference in the catalytic activities of the enzymes were compared according to the ratio of the grayscale values of the liner ligation product band to the 3'-OH end substrate band as catalyzed different mutant enzymes. The results are shown in Figure 6.

The linear ligation reaction test results showed that the enzyme activity was significantly improved after any of the four amino acids of R79, R281, K370, and K372 was mutated to alanine, or both the K370 and K372 were mutated to alanine. At the same time, for K370 and K372 positions, mutation of lysine to other amino acids improved the catalytic activity of the enzyme.

Furthermore, when the K370 and K372 sites were mutated to other amino acids, the results showed that the activity was significantly improved compared to wild-type HyperLigase after mutating these two sites to any other amino acids (Figures 5 and 6).

For the activity test of the above mutation sites, the linear ligation reaction results were consistent with the single-stranded DNA circularization reaction results.

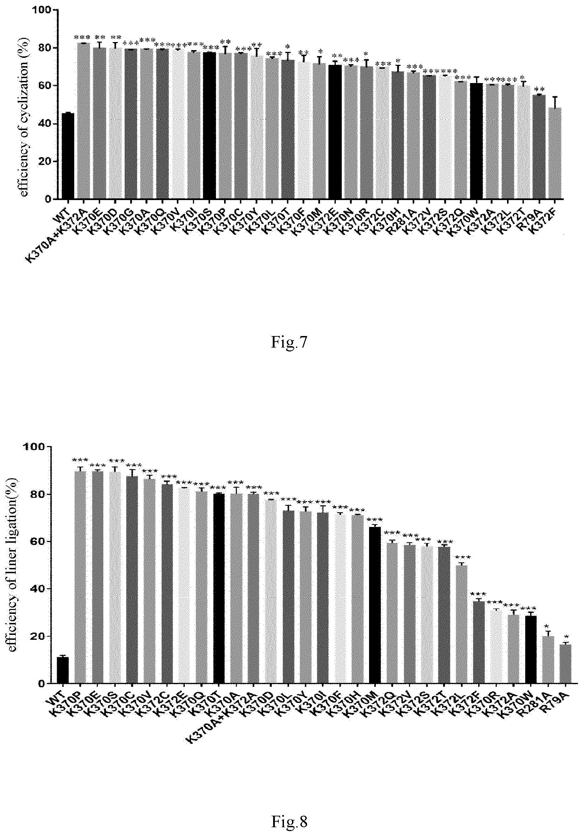

In summary, the mutations for any of the R79, R281, K370, and K372 residues or the combined mutations of K370 and K372 significantly improve the activity of the enzyme. Additionally, for the K370 and K372 positions, mutation of lysine to other amino acids improved the catalytic activity of the enzyme (see Figures 7 and 8).

### Example 5

### Test of the activity of K370P mutant ligase in ligating the 3'-hydroxyl end of RNA and the 5' adenylated end of DNA

A 21-base RNA sequence was selected as the acceptor, and a 35-base 5'-end pre-adenylated and 3'-end blocked DNA was selected as the donor. It was expected that the 3' hydroxyl end of the RNA was ligated to the 5' adenylated end of the DNA. The reaction system was as follows:

| reagent | Initial concentration | Final concentration | 1× (µL) |
|---|---|---|---|
| Tris-HCl, pH=8 | 700mM | 70 mM | 2 |
| 5'-rApp-CL78_cP5_UMI8 | 5 µM | 0.25 µM | 1 |
| 5P-21mer-RNA | 1 µM | 0.25µM | 5 |
| PEG8000 | 50% w/v | 20% | 8 |
| H₂O | / | / | 1.74 |
| Hyperligase K370P or WT | 3.98µM | 0.25µM | 1.26 |
| MnCl₂ | 100mM | 5mM | 1 |
| total | | / | 20 |

Reaction conditions: 65°C, 30min/1h; 4°C, hold. The reaction products were identified by Urea-PAGE electrophoresis.

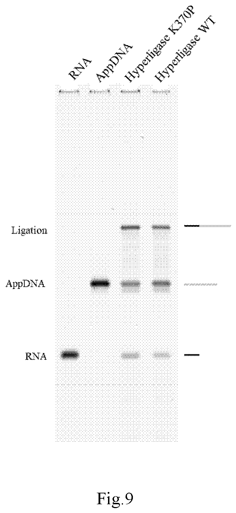

The results are shown in Figure 9, which shows that the K370P mutation enhanced the activity of Hyperligase on the ligation between the 3' hydroxyl end of RNA and the 5' adenylylated end of

DNA. It can be seen from the figure that the grayscale of the band of the K370P ligation product increased, and the substrate band of AppDNA was weaker than the wild-type, thus indicating the ligation activity of the enzyme was improved.

### Example 7

### Test of the activity of K370P mutant ligase in ligating the 3'-hydroxyl end of DNA and the 5' adenylated end of RNA

A 28-base RNA sequence was selected as the donor, and a 55-base single-stranded DNA with a hydroxyl group at the 3' end was selected as the acceptor. It was expected that the 3' hydroxyl end of the DNA was ligated to the 5' adenylated end of the RNA. The reaction system was as follows:

| reagent | Initial concentration | Final concentration | 1× (µL) |
|---|---|---|---|
| H₂O | | / | 5.9 |
| Tris-HCl, pH=8 | 700mM | 70 mM | 2 |
| 5App3NH2-28mer-RNA | 3.73 | 0.25 | 1.34 |
| OH-5N3N-Cir-Con | 10 µM | 0.5µM | 0.5 |
| PEG8000 | 50% w/v | 20% | 8 |
| Hyperligase K370P or WT | 3.98µM | 0.25µM | 1.26 |
| MnCl₂ | 100mM | 5mM | 1 |
| total | | / | 20 |

Reaction conditions: 65°C, 30min; 16°C, hold.

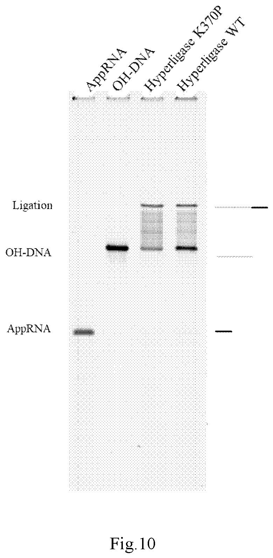

The results are shown in Figure 10, which shows that the K370P mutation enhanced the activity of Hyperligase on the ligation between the 3' hydroxyl end of DNA and the 5' adenylated end of RNA. It can be clearly seen that the amount of OH-DNA substrate of K370P was reduced, indicating that more substrates were used for ligation. There was degradation in the ligation product, with stronger degradation bands derived from K370P ligase than from WT HyperlLigase, also confirming better ligation efficiency of the former.

### references:

[1] VIOLLET S, FUCHS R T, MUNAFO D B, et al. T4 RNA ligase 2 truncated active site mutants: improved tools for RNA analysis [J]. BMC Biotechnol, 2011, 11(72.
[2] LOHMAN G J, TABOR S, NICHOLS N M. DNA ligases [J]. Curr Protoc Mol Biol, 2011, Chapter 3(Unit3 14.
[3] JOHNSON A, O'DONNELL M. DNA ligase: Getting a grip to seal the deal [J]. Current Biology, 2005, 15(3): R90-R2.
[4] CHEREPANOV A V, DE VRIES S. Dynamic mechanism of nick recognition by DNA ligase [J]. European Journal of Biochemistry, 2002, 269(24): 5993-9.
[5] TUMBALE P P, JURKIW T J, SCHELLENBERG M J, et al. Two-tiered enforcement of high-fidelity DNA ligation [J]. Nature Communications, 2019, 10(1): 5431.
[6] FOY C A, PARKES H C. Emerging Homogeneous DNA-based Technologies in the Clinical Laboratory [J]. Clinical Chemistry, 2001, 47(6): 990-1000.
[7] BLONDAL T, THORISDOTTIR A, UNNSTEINSDOTTIR U, et al. Isolation and characterization of a thermostable RNA ligase 1 from a Thermus scotoductus bacteriophage TS2126 with good single-stranded DNA ligation properties [J]. Nucleic Acids Res, 2005, 33(1): 135-42.
[8] ZHELKOVSKY A M, MCREYNOLDS L A. Structure-function analysis of Methanobacterium thermoautotrophicum RNA ligase - engineering a thermostable ATP independent enzyme [J]. BMC Molecular Biology, 2012, 13(1): 10.

## Claims

1. A nucleic acid ligase, which comprises an amino acid sequence having a mutation at one or more positions selected from positions 79, 281, 370 and 372 compared to the amino acid sequence of SEQ ID NO: 1.

2. The nucleic acid ligase of claim 1, wherein the mutation at position 79 is the substitution of Arg with Ala, the mutation at position 281 is the substitution of Arg with Ala, the mutation at position 370 is the substitution of Lys with any natural amino acid except Lys, and the mutation at position 372 is the substitution of Lys with any natural amino acid except Lys.

3. The nucleic acid ligase of claim 1, which comprises an amino acid sequence selected from SEQ ID NOs: 2-7.

4. The nucleic acid ligase of claim 3, which comprises the amino acid sequence of SEQ ID NO: 2 or 3, wherein Xaa is a non-polar amino acid, preferably Ala; or, which comprises the amino acid sequence of SEQ ID NO: 4 or 5.

5. The nucleic acid ligase of claim 3, comprising the amino acid sequence of SEQ ID NO: 6.

6. The nucleic acid ligase of claim 5, wherein Xaa at positions 370 and 372 is Ala.

7. The nucleic acid ligase of claim 3, which comprises the amino acid sequence of SEQ ID NO: 7, wherein Xaa at position 79 or 281 is a non-polar amino acid, preferably Ala.

8. The nucleic acid ligase of claim 7, wherein Xaa at positions 370 and 372 is a non-polar amino acid, preferably Ala.

9. A nucleic acid molecule encoding the nucleic acid ligase of any one of claims 1-8.

10. A vector comprising the nucleic acid molecule of claim 9.

11. A recombinant cell into which the nucleic acid of claim 9 or the vector of claim 10 has been introduced.

12. A composition for ligating single-stranded DNAs and/or RNAs, comprising the nucleic acid ligase of any one of claims 1-8.

13. A kit for ligating single-stranded DNAs and/or RNAs, comprising the nucleic acid ligase of any one of claims 1-8.

14. Use of the nucleic acid ligase of any one of claims 1 to 8 in the preparation of a product for ligating single-stranded DNAs and/or RNAs.
